# EUROPÄISCHE PATENTSCHRIFT

(11) **EP 1 015 447 B1**
(45) Veröffentlichungstag und Bekanntmachung des Hinweises auf die Patenterteilung: **10.04.2002**
(21) Anmeldenummer: 98945288.3
(22) Anmeldetag: 26.08.1998
(51) Int. Cl.: C07D 413/06, A61K 31/42

(54) **PIPERIDINYLMETHYLOXAZOLIDINON-DERIVAT**
PIPERIDINYLMETHYLOXAZOLIDINONE DERIVATIVE
DERIVE DE PIPERIDINYLMETHYLOXAZOLIDINONE

(30) Priorität: 09.09.1997 DE 19739332
(43) Veröffentlichungstag der Anmeldung: 05.07.2000
(73) Patentinhaber: MERCK PATENT GmbH, 64293 Darmstadt (DE)
(72) Erfinder: PRÜCHER, Helmut, D-64646 Heppenheim (DE); BARTOSZYK, Gerd, D-64331 Weiterstadt (DE); LEIBROCK, Joachim, D-64319 Pfungstadt (DE); SEYFRIED, Christoph, D-64342 Seeheim-Jugenheim (DE); GOTTSCHLICH, Rudolf, D-64354 Reinheim (DE)
(86) Internationale Anmeldenummer: EP9805402
(87) Internationale Veröffentlichungsnummer: WO9912924

(56) Entgegenhaltungen:
- EP-A- 0 763 535

## Beschreibung

Die Erfindung betrifft die Verbindung 5-[4-(4-Fluorobenzyl)-piperidin-1-ylmethyl]-3-(4-hydroxyphenyl)-oxazolidin-2-on der Formel I 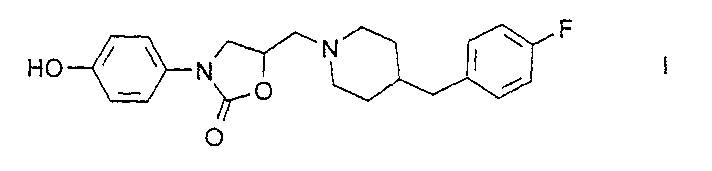 sowie deren physiologisch unbedenklichen Salze.

Gegenstand der Erfindung ist die Verbindung der Formel I gemäß Anspruch I sowie deren Enantiomere und deren Salze.

Piperidinylmethyloxazolidon-Derivate als psychopharmakologisch wirksame Substanzen sind aus der EP 0 763 535 bekannt.
In Bezug auf dieses Schutzrecht ist die erfindungsgemäße Verbindung als Auswahlerfindung anzusehen.

Der Erfindung lag die Aufgabe zugrunde, neue Verbindungen zur Verfügung zu stellen, die zur Herstellung von Arzneimitteln verwendet werden können, jedoch gegenüber den bereits bekannten Wirkstoffen ein ausgeprägteres Wirkspektrum aufweisen und selektiv auf das Zentralnervensystem wirken, nebenwirkungsarm sind, gleichzeitig aufgrund einer geänderten Struktur in möglichst geringer Dosis verabreicht werden können und dabei kein oder nur ein sehr geringes Abhängigkeitspotential aufweisen.

Es wurde nun gefunden, daß die Verbindung der Formel I und ihre physiologisch unbedenklichen Salze bei guter Verträglichkeit wertvolle pharmakologische Eigenschaften besitzt. Sie beeinflußt insbesondere das Zentralnervensystem und weist neuroleptische, tranquillierende, anxiolytische, antidepressive und gedächtnisfördernde Wirkungen auf.

Gegenstand der Erfindung ist daher die Verbindung der gegebenen Formel I sowie ihre Salze und deren Verwendung als pharmakologisch wirksame Substanz.

Gegenstand der Erfindung sind aber auch geeignete Verfahren zur Herstellung dieser Verbindungen bzw. deren Salze.

Im einzelnen hat die Verbindung der gegebenen Formel I sowie ihre Salze eine neuroleptische Wirkung; sie hemmt das durch Apomorphin induzierte Kletterverhalten bei Mäusen, die durch Apomorphin induzierten Stereotypien bei Ratten (Methodik vgl. Costall et al., European J. Pharmacol. 50 (1978), 39-50, bzw. Puech et al., European J. Pharmacol. 50 (1978) 291-300) und die konditionierte Vermeidungsreaktion bei Ratten (Methodik vgl. Niemegeers et al., Psychopharmacology 16 (1969), 161-174), ohne daß Katalepsie auftritt (Methodik vgl. Stanley und Glick, Neuropharmacology 15 (1976), 393-394), was als Hinweis auf ein fehlendes Nebenwirkungspotential bezüglich extrapyramidal-motorischer Nebenwirkungen gewertet wird (Hoffmann und Donovan, Psychopharmacology 120 (1995), 128-133). Sie hemmt die Ultraschallvokalisation nach elektrischer Stimulation bei Ratten (Nachweis der anxiolytischen Wirkung; Methodik vgl. De Vry et al., European J. Pharmacol. 249 (1993), 331-339) und hat eine dämpfende Wirkung auf das Spontanverhalten von Mäusen und Ratten (Methodik vgl. Irwin, Psychopharmacology 13 (1968), 222-257). Auch verdrängt dieser Wirkstoff im Bindungsexperiment tritiiertes Ifenprodil im Vorderhirn der Ratte von seiner Bindungsstelle (Methodik vgl. Schoemaker et al., European J. Pharmacol. 176 (1990), 249-250), die einen Rezeptor am N-Methyl-D-Aspartat (NMDA) Rezeptor-lonenkanal-Komplex als Untertyp der Glutamat-Rezeptoren darstellt.

Aufgrund der Glutamat-Mangelhypothese der Schizophrenie (Ishimaru und Toru, CNS Drugs 7 (1997), 47-67; Carlsson et al., Int. Acad. Biomed. Drug Res., 4 (1993), 118) stellen Substanzen, die eine agonistische Wirkung an Glutamat-Rezeptoren entfalten, ein völlig neues Wirkungsprinzip zur Behandlung der Schizophrenie dar, während im Gegensatz dazu übliche Neuroleptika direkt als Antagonisten am Dopamin-Rezeptor wirken (gemäß der klassischen Dopamin-Überfunktions-Hypothese der Schizophrenie, Carlsson et al., Life Sciences 61 (1997), 75-94) mit dem Nachteil, daß sie die typischen extrapyramidal-motorischen Nebenwirkungen hervorrufen, die nach Langzeitbehandlung z.T. irreversibel sind, und mentale Beeinträchtigungen wie Angst induzieren (Casey, Int. Clinical Psychopharmacology 10 Suppl. 3 (1995), 105-114).

Überraschenderweise wurde nun gefunden, daß das 5S-Enantiomer der erfindungsgemäßen Verbindung in vitro ein potenter Ligand im nanomolaren Konzentrationsbereich für die Polyamin-Bindungsstelle ist und neuroleptische Wirkungen im Vergleich zu den Verbindungen der Formel A, B und C (C s. EP 0 763 535; Seite 9, Zeile 18) aufweist.

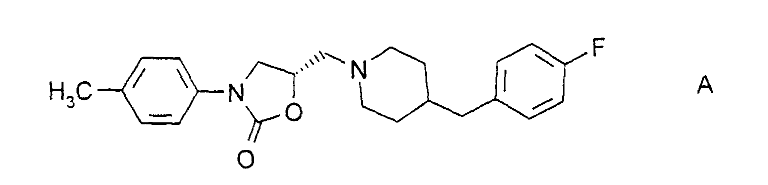

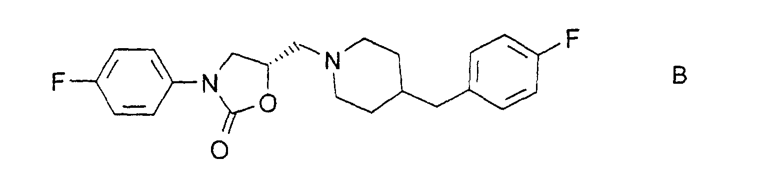

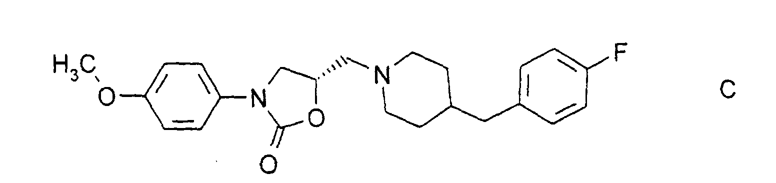

Die pharmakologischen Testdaten sind in Tabelle I zusammen gefaßt.

Aufgrund dieser Ergebnisse der Untersuchungen hat sich gezeigt, daß die Verbindung der Formel I sowie ihre physiologisch unbedenklichen Säureadditionssalze als Arzneimittelwirkstoff aber auch als Zwischenprodukt zur Herstellung weiterer Arzneimittelwirkstoffe verwendet werden kann.

Gegenstand der Erfindung ist ferner ein Verfahren zur Herstellung der Verbindung der Formel I gemäß Anspruch 1, dadurch gekennzeichnet, daß man
a) eine Verbindung der Formel II 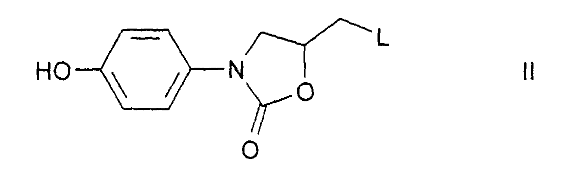 worin
   L Cl, Br, I oder eine freie oder reaktionsfähig funktionell abgewandelte OH-Gruppe bedeutet,
   mit einer Verbindung der Formel III 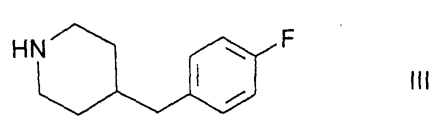 umsetzt,
   oder
b) eine Verbindung der Formel IV 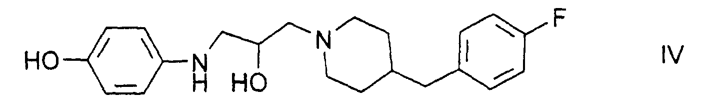 mit einer Verbindung der Formel V 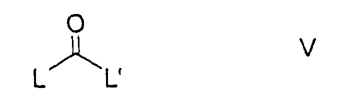 worin L und L' jeweils unabhängig voneinander Cl, Br, I oder eine freie oder reaktionsfähig funktionell abgewandelte OH-Gruppe bedeutet, umsetzt,
   oder
c) eine Verbindung der Formel VI 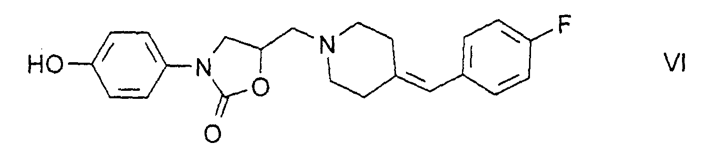 hydriert,
   oder
d) die Verbindung der Formel I aus einem ihrer funktionellen Derivate durch Behandeln mit einem solvolysierenden oder hydrogenolysierenden Mittel in Freiheit setzt,
und/oder daß man eine basische Verbindung der Formel I durch Behandeln mit einer Säure in eines ihrer Salze überführt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag; J. March, Advanced Organic Chemistry 3rd. Ed. (1984) oder Organic Reactions, beide John Wiley & Sons, Inc. New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Verbindung der Formel I kann vorzugsweise erhalten werden, indem man eine Verbindung der Formel II mit der Verbindung der Formel III umsetzt.

Die Ausgangsstoffe der Formeln II und III sind teilweise bekannt. Sofern sie nicht bekannt sind, können sie nach an sich bekannten Methoden hergestellt werden.

Primäre Alkohole der Formeln II sind z.B. durch Reduktion der entsprechenden Carbonsäuren oder ihrer Ester erhältlich. Behandlung mit Thionylchlorid, Bromwasserstoff, Phosphortribromid oder ähnlichen Halogenverbindungen liefert die entsprechenden Halogenide der Formel II.

Die Verbindung der Formel III kann beispielsweise analog Schema 1 hergestellt werden.

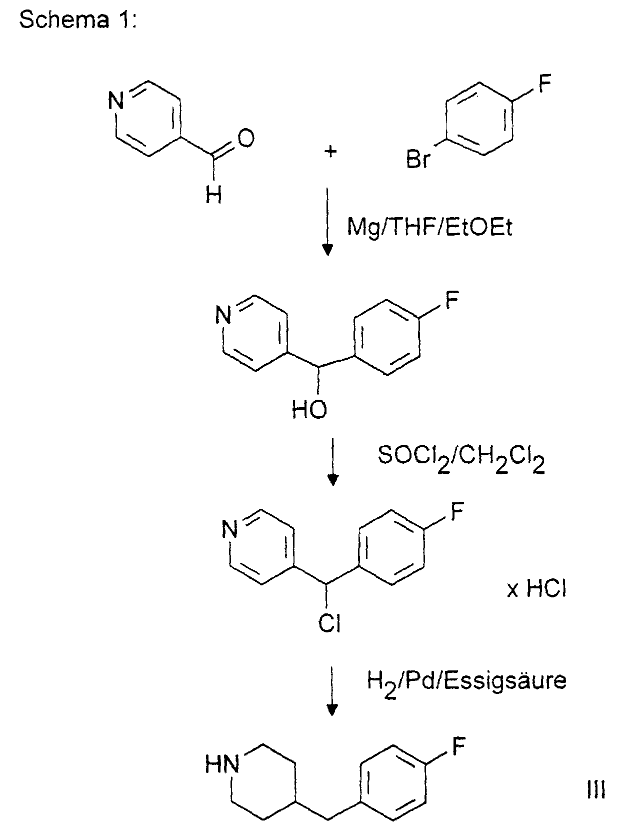

Die Umsetzung der Verbindungen der Formel II mit der Verbindung der Formel III erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Sulfonyloxyverbindungen der Formel II sind aus den entsprechenden Alkoholen durch Umsetzung mit den entsprechenden Sulfonsäurechloriden erhältlich. Die lodverbindungen der Formel II sind z. B. durch Einwirkung von Kaliumjodid auf die zugehörigen p-Toluolsulfonsäureester erhältlich.

Verbindungen der Formel I lassen sich weiterhin bevorzugt durch Umsetzung von Verbindungen der Formel IV mit Verbindungen der Formel V erhalten. Als Verbindungen der Formel V eignen sich bevorzugt Dialkylcarbonate wie Dimethyl-, Ditrichlormethyl- oder Diethylcarbonat, Chlorameisensäureester wie Chlorameisensäuremethyl- oder -ethylester, N,N'-Carbonyldiimidazol oder Phosgen.

Die Ausgangsstoffe der Formeln IV und V sind teilweise bekannt. Sofern sie nicht bekannt sind, können sie nach an sich bekannten Methoden hergestellt werden. Die Umsetzung erfolgt in Lösungsmitteln und bei Temperaturen wie oben beschrieben.

Die Verbindung der Formel VI läßt sich weiterhin reduktiv in die Verbindunge der Formel I überführen. Vorzugsweise bedient man sich hierzu der katalytischen Hydrierung mit z.B. Palladium auf Aktivkohle und Wasserstoff.

Es ist ferner möglich, eine Verbindung der Formel I zu erhalten, indem man ein Vorprodukt, das anstelle von Wasserstoffatomen eine oder mehrere reduzierbare Gruppe(n) und /oder eine oder mehrere zusätzliche C-Cund/oder C-N-Bindung(en) enthält, mit einem reduzierbaren Mittel behandelt, vorzugsweise bei Temperaturen zwischen -80 und 250 °C in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. Toluolsulfonyloxy), N-Benzolsulfonyl, N-Benzyl oder O-Benzyl.

Es ist grundsätzlich möglich, Verbindungen, die nur eine oder solche, die nebeneinander zwei oder mehrere dieser Gruppen bzw. zusätzlichen Bindungen enthalten, reduktiv in eine Verbindung der Formel I zu überführen. Vorzugsweise bedient man sich hierzu der katalytischen Hydrierung des nascierenden Wasserstoffs oder bestimmter komplexer Metallhydride wie NaBH₄ oder LiAlH₄.

Für die katalytische Hydrierung sind als Katalysatoren beispielsweise Edelmetall-, Nickel und Kobaltkatalysatoren geeignet. Die Edelmetallkatalysatoren können auf Trägern (z. B. Platin oder Palladium auf Kohle, Palladium auf Calciumcarbonat oder Strontiumcarbonat), Oxidkatalysatoren (z. B. Platinoxid), oder als feinteilige Metallkatalysatoren vorliegen. Nickel- und Kobaltkatalysatoren werden zweckmäßig als Raney-Metalle, Nickel auch auf Kieselgur oder Bimsstein als Träger eingesetzt. Die Hydrierung kann bei Raumtemperatur und Normaldruck oder auch bei erhöhter Temperatur und/oder erhöhtem Druck durchgeführt werden. Vorzugsweise arbeitet man bei Drucken zwischen 1 und 100 bar und bei Temperaturen zwischen -80 und +150 °C, in erster Linie zwischen Raumtemperatur und 100 °C. Die Umsetzung wird zweckmäßig im sauren, neutralen oder basischen Bereich und in Gegenwart eines Lösungsmittels, wie Wasser, Methanol, Ethanol, Isopropanol, n-Butanol, Ethylacetat, Dioxan, Essigsäure oder THF durchgeführt, man kann auch Gemische dieser Lösungsmittel einsetzen.

Wird als Reduktionsmittel nascierender Wasserstoff verwendet, so kann man diesen z. B. durch Behandeln von Metallen mit schwachen Säuren oder mit Basen erzeugen. So kann man z. B. ein Gemisch aus Zink und Alkalilauge oder aus Eisen und Essigsäure verwenden. Geeignet ist auch die Verwendung von Natrium oder eines anderen Alkalimetalls in einem Alkohol wie Ethanol, Isopropanol, Butanol, Amyl- oder Isoamylalkohol oder Phenol. Man kann ferner eine Aluminium-Nickel-Legierung in alkalischwäßriger Lösung, gegebenenfalls unter Zusatz von Ethanol verwenden. Auch Natrium- oder Aluminiumamalgam in wäßrig-alkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner komplexe Metallhydride, wie NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether, wie Diethylether, Di-n-butylether, THF, Dioxan, Diglyme oder 1,2-Dimethoxyethan, sowie Kohlenwasserstoffe, wie Benzol. Für eine Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen - 80 und +150 °C, insbesondere zwischen 0 und etwa 100 °C.

Die Verbindungen der Formel I können auch erhalten werden, indem man sie aus ihren funktionellen Derivaten durch Solvolyse, insbesondere durch Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Hydroxygruppen entsprechende geschützte Hydroxygruppen enthalten, vorzugsweise solche, die an Stelle des H-Atoms einer Hydroxygruppe eine Hydroxyschutzgruppe tragen.

Der Ausdruck "Hydroxyschutzgruppe" ist allgemein bekannt und bezieht sich auf Gruppen, die geeignet sind, eine Hydroxygruppe vor chemischen Umsetzungen zu schützen, die aber leicht entfernbar sind, nachdem die gewünschte Reaktion an der anderen Stelle des Moleküls erfolgt ist. Typisch für solche Gruppen sind die oben genannten unsubstituierten oder substituierten Aryl-, Aralkyl- oder Acylgruppen, ferner auch Alkylgruppen. Auch in diesem Fall ist die Natur und Größe der Hydroxyschutzgruppen nicht kritisch, da sie nach der gewünschten chemischen Reaktion oder Reaktionsfolge wieder entfernt werden. Es werden jedoch Schutzgruppen mit 1-20, insbesondere mit 1-10 C-Atomen bevorzugt. Beispiele für solche Hydroxyschutzgruppen sind u. a. tert.-Butyl, Benzyl, p-Nitrobenzoyl, p-Toluolsufonyl und Acetyl, wobei Benzyl und Acetyl besonders bevorzugt sind.

Das In-Freiheit-Setzen der Verbindungen der Formel I aus ihren funktionellen Derivaten gelingt, je nach der benutzten Schutzgruppe - z.B. mit starken Säuren wie Salzsäure oder Schwefelsäure, starken Carbonsäuren wie Trichloressigsäure oder Sulfonsäuren wie Benzol- oder p-Toluolsulfonsäure. Dieses kann, falls erforderlich, in Anwesenheit eines zusätzlichen Lösungsmittels erfolgen.

Als inerte Lösungsmittel eignen sich für diesen Zweck insbesondere organische Lösungsmittel, und zwar Carbonsäuren wie Essigsäure, Ether wie Tetrahydrofuran, Amide wie Dimethylformamid, halogenierte Kohlenwasserstoffe wie Dichlormethan, ferner auch Alkohole wie Methanol, Ethanol oder Isopropanol sowie Wasser. Es kommen aber auch Gemische dieser Lösungsmittel in Frage. Für diesem Zweck werden bevorzugt physiologisch verträgliche, inerte Lösungsmittel ausgewählt, bzw solche, die falls geringste Reste im hergestellten Produkt verbleiben sollten, kein gesundheitliches Risiko darstellen.

Trifluoressigsäure wird vorzugsweise im Überschuß ohne Zusatz eines weiteren Lösungsmittels verwendet. Perchlorsäure wird dagegen in einer Mischung aus Essigsäure und 70%iger Perchlorsäure im Verhältnis 9:1 verwendet. Die Abspaltung der Schutzgruppen erfolgt zweckmäßigerweise bei Temperaturen von etwa 0-50 °C, vorzugsweise bei 15-30 °C bzw. Raumtemperatur.

Tert.-Butoxycarbonyl wird bevorzugt mit 40%iger Trifluoressigsäure in Dichlormethan oder, falls es nicht anders durchführbar ist, mit etwa 3 bis 5 n HCI in Dioxan bei 15 - 60 °C abgespalten werden. 9-Fluorenylmethoxycarbonyl-Gruppen werden mit einer etwa 5-20%igen Lösung von Dimethylamin, Diethylamin oder Piperidin in DMF bei 15-50 °C abgespalten. Eine Abspaltung von 2,4-Dinitrophenylgruppen gelingt mit einer etwa 3-10%igen Lösung von 2-Mercaptoethanol in DMF/Wasser bei 15-30 °C.

Hydrogenolytisch entfernbare Schutzgruppen, wie Benzyloxymethyl, Benzyloxycarbonyl oder Benzyl, können durch Behandeln mit Wasserstoff in Gegenwart eines Katalysators (z. B. Edelmetallkatalysators wie Palladium, zweckmäßig auf einem Träger wie Kohle) abgespalten werden. Als Lösungsmittel eignen sich dafür die oben angegebenen, insbesondere Alkohole wie Methanol oder Ethanol oder Amide wie DMF. Die Hydrogenolyse wird in der Regel bei Temperaturen zwischen 0 und 100 °C und Drucken zwischen 1 und 200 bar, vorzugsweise bei 20-30 °C und 1-10 bar durchgeführt. Eine Hydrogenolyse von Benzyloxycarbonylgruppen gelingt beispielsweise gut an 5-10%igem Pd-C in Methanol bei 20 bis 30 °C.

Weiterhin kann man gegebenenfalls eine Verbindung der Formel I nach an sich bekannten Methoden in eine andere Verbindung der Formel I umwandeln.

So können entsprechende Ether gespalten werden, wobei die korrespondierenden Hydroxyderivate entstehen. Solche Ether können auch durch Behandeln mit einem Dimethylsulfid-Bortribromid-Komplex in einem Lösungsmittel wie Toluol, 1,2-Dichlorethan, THF oder Dimethylsulfoxid gespalten werden oder durch Verschmelzen mit Pyridin- oder Anilinhydrohalogeniden. Vorzugsweise wird diese Reaktion mit Pyridinhydrochlorid bei etwa 150-250°C, mit HBr/Essigsäure oder mit AI-trihalogeniden in chlorierten Kohlenwasserstoffen wie 1,2-Dichlorethan durchgeführt.

Die Verbindung der Formel I besitzt ein Asymmetriezentrum. Sie kann daher als Racemat oder, falls eine optisch aktive Ausgangsverbindung eingesetzt wird, auch in optisch aktiver Form erhalten werden. Ein erhaltenes Racemat kann, falls erwünscht, nach an sich bekannten Methoden physikalisch oder chemisch aufgetrennt werden. Vorzugsweise werden aus Racematen durch chemische Umsetzung mit einem optisch aktiven Trennmittel Diastereomere gebildet. Als Trennmittel eignen sich z. B. optisch aktive Säuren, wie die D- und L-Formen der Weinsäure, Dibenzoylweinsäure, Diacetylweinsäure, Camphersulfonsäure, Mandelsäure, Äpfelsäure oder Milchsäure. Die verschiedenen Formen der Diastereomere können in an sich bekannter Weise, z. B. durch fraktionierte Kristallisation, getrennt werden und die optisch aktiven Verbindungen der Formel I in an sich bekannter Weise aus den Diastereomeren in Freiheit gesetzt werden.

Eine erhaltene Base der Formel I kann mit einer Säure in das dazugehörige Säureadditionssalz überführt werden. Hierzu eignen sich insbesondere Säuren, die physiologisch unbedenkliche Salze liefern. Als anorganische Säuren können zu diesem Zweck Schwefelsäure, Halogenwasserstoffsäuren wie HCI, HBr, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure, Säureadditionssalze, die physiologisch nicht unbedenklich sind, können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der allgemeinen Formel I und ihre physiologisch unbedenklichen Salze können daher zur Herstellung pharmazeutischer Präparate verwendet werden, indem man sie zusammen mit mindestens einem Träger- oder Hilfsstoff und, falls erwünscht, mit einem oder mehreren weiteren Wirkstoffen in die geeignete Dosierungsform bringt.

Die so erhaltenen Zubereitungen können als Arzneimittel in der Humanoder Veterinärmedizin eingesetzt werden.

Als Trägersubstanzen kommen organische oder anorganische Stoffe in Frage, die sich für die enterale (z. B. orale oder rektale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Cellulose, Magnesiumstearat, Talk oder Vaseline, Glycerintriacetat und andere Fettsäureglyceride, Sojalecithin.

Zur oralen Anwendung dienen insbesondere Tabletten, Dragees, Kapseln, Sirupe, Säfte oder Tropfen. Von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wäßrige Lösungen, ferner Suspensionen, Emulsionen oder Implantate, sowie für die topische Anwendung Salben, Cremes oder Puder.

Die erfindungsgemäß beanspruchten Wirkstoffe können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Die Verbindung der Formel I und ihre physiologisch unbedenklichen Salze kann zur therapeutischen Behandlung des menschlichen oder tierischen Körpers, insbesondere zur Bekämpfung von Krankheiten verwendet werden. Sie ist in der Behandlung der Schizophrenie und von affektiven Störungen wie z.B. Depression und/oder Angst wirksam. Auch kann die Verbindung bei der Behandlung extrapyramidaler Störungen Anwendung finden. Die erfindungsgemäße Verbindung ist als Neuroleptikum wirksam, zeigt dabei jedoch in vorteilhafter Weise keine nennenswerten kataleptischen Nebenwirkungen auf.
Die Verbindung ist ferner wirksam zur Behandlung von Schlaganfall.

Die erfindungsgemäße Verbindung gemäß Formel I und ihre physiologisch unbedenklichen Salze werden in der Regel in Analogie zu anderen bekannten, für die beanspruchten Indikationen im Handel erhältlichen Präparaten (Thioridazin, Haloperidol) verabreicht, vorzugsweise in Dosierungen zwischen etwa 0,1 mg und 500 mg, insbesondere zwischen 0,2 und 50 mg pro Dosierungseinheit. Die tägliche Dosierung liegt vorzugsweise zwischen etwa 0.002 und 20 mg/kg, insbesondere 0,2 und 0,4 mg/kg Körpergewicht.

Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.
Im folgenden werden Beispiele gegebenen, die zur Veranschaulichung der Erfindung dienen, jedoch die Erfindung nicht auf die gegebenen Beispiele begrenzen.

Die erfindungsgemäße Verbindung der Formel I kann aufgrund ihrer Molekülstruktur in zwei enantiomeren Formen auftreten. Sie kann daher in racemischer oder in optisch aktiver Form vorliegen.

Da sich die pharmazeutische Wirksamkeit der Racemate bzw. der Stereoisomeren der erfindungsgemäßen Verbindung unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Salze mit physiologisch nicht unbedenklichen Säuren, z. B. Pikrate, können zur Isolierung und/oder Aufreinigung der Verbindungen der Formel I verwendet werden.

In den nachstehenden Beispielen bedeutet "übliche" Aufarbeitung: Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Alle Temperaturen sind in °C angegeben und die [α]_{D}-Werte bei 20 °C in Dimethylsulfoxid gemessen.

### Beispiel 1

Eine Lösung, bestehend aus 4,92 g (5R)-(-)-5-(Methansulfonsäureoxymethyl)-3-p-hydroxyphenyl-oxazolidin-2-on [erhältlich aus (5R)-(-)-5-(Methansulfonsäure-oxymethyl)-3-p-methoxyphenyl-oxazolidin-2-on durch Behandeln mit Bortribromid in Dichlormethan], 65 ml Acetonitril, 4,70 g 4-(4-Fluorbenzyl)-piperidinhydrochlorid und 4,43 g Natriumhydrogencarbonat, wird für die Dauer von 26 Stunden unter Rückflußbedingungen gerührt. Anschließend wird das Reaktionsgemisch mit 100 ml Dichlormethan verdünnt, mehrmals mit geringen Mengen Wasser extrahiert, getrocknet. Nach dem Trocknen wird das Lösungsmittel abdestilliert und das erhaltene Produkt chromatographisch an einer Kieselgelsäule gereinigt. Auf diese Weise wird das Reaktionsprodukt als farbloses Harz erhalten, das auskristallisiert wird.
Ausbeute: (5S)-(-)-5-[4-(4-Fluorbenzyl)-1-piperidinylmethyl]-3-(4-hydroxyphenyl)-oxazolidin-2-on
Fp. 164-165°
[α]_{D}²⁰ = -28,0°

Durch Umsetzung mit Methansulfonsäure in Aceton erhält man 3,18 g (5S)-(-)-5-[4-(4-Fluorbenzyl)-1-piperidinylmethyl]-3-(4-hydroxyphenyl)-oxazolidin-2-on, Methansulfonat
Fp. 234-236°
[α]_{D}²⁰ = -33,3°.

### Beispiel 2

Eine Lösung von 1 g (5S)-(-)-5-[4-(4-Fluorbenzyl)-1-piperidinylmethyl]-3-(4-benzyloxyphenyl)-oxazolidin-2-on [erhältlich durch Umsetzung von (5R)-(-)-5-(Methansulfonsäure-oxymethyl)-3-p-benzyloxyphenyl-oxazolidin-2-on mit 4-(4-Fluorbenzyl)-piperidinhydrochlorid] in 25 ml Methanol wird bei Normaldruck und 20° C an 1 g Raney-Nickel hydriert. Man filtriert, entfernt das Lösungsmittel und enthält (5S)-(-)-5-[4-(4-Fluorbenzyl)-1-piperidinylmethyl]-3-(4-hydroxyphenyl)-oxazolidin-2-on,
Fp. 164-165°
[α]_{D}²⁰ = -28,0°.

### Beispiel 3

Eine Lösung von 1 g (5S)-(-)-5-[4-(4-Fluorbenzyl)-1-piperidinylmethyl]-3-(4-methoxyphenyl)-oxazolidin-2-on [erhältlich durch Umsetzung von (5R)-(-)-5-(Methansulfonsäure-oxymethyl)-3-p-methoxyphenyl-oxazolidin-2-on mit 4-(4-Fluorbenzyl)-piperidinhydrochlorid] in 25 ml Dichlormethan wird mit äquimolaren Mengen Bortribromid versetzt und 1 Stunde nachgerührt. Nach üblicher Aufarbeitung erhält man (5S)-(-)-5-[4-(4-Fluorbenzyl)-1-piperidinylmethyl]-3-(4-hydroxyphenyl)-oxazolidin-2-on,
Fp. 164-165°
[α]_{D}²⁰ = -28,0°.

### Pharmakologische Tests

1. Ifenprodil-Bindung (Schoemaker et al., 1990):
   Homogenate vom Ratten-Vorderhirn wurden mehrfach zentrifugiert. Mit dem letzten Membranen-Überstand (10 mg/ml) wurde die spezifische Bindung in Gegenwart von 1,15 nM [³H]-Ifenprodil in 5 ml Pufferlösung bei pH 7,4 bestimmt. Die unspezifische Bindung wurde in der Gegenwart von 100 µM Ifenprodil bestimmt.
2. Hemmung Apomorphin-induzierter Stereotypien bei ratten (Puech et al., 1978):
   Stereotypien (unmotiviertes Schnüffeln, Lecken, Kauen, rhythmische bewegungen mit den Vorderpfoten) wurden durch die Gabe von 0,5 mg/kg s.c. Apomorphin induziert. Die Stärke der auftretenden Stereotypien wurde für jede Ratte alle 5 Minuten für die Dauer von 30 Minuten nach einem Ratingsystem (0-3) bewertet. Der Summenscore diente als Maß für das Auftreten der Stereotypien.
3. Katalepsie-Induktion bei Ratten (Stanley und Glick, 1976):
   Das Auftreten von Katalepsie bei der Ratte wurde gemessen, indem eine Hinterpfote auf einen 3 cm hohen Block plaziert und alle 5 Minuten für die Dauer von 30 Minuten nach einem Ratingsystem (Score 0-3) die Zeit bewertet wurde, die die Ratte in dieser unnatürlichen Stellung verharrte. Der Summenscore diente als Maß für die Stärke der Katalepsie.

Die Ergebnisse der Tests mit dem 5S-Enantiomer der erfindungsgemäßen Verbindung und der Vergleichsverbindungen A, B und C sind in der nachfolgenden Tabelle I zusammengefaßt.

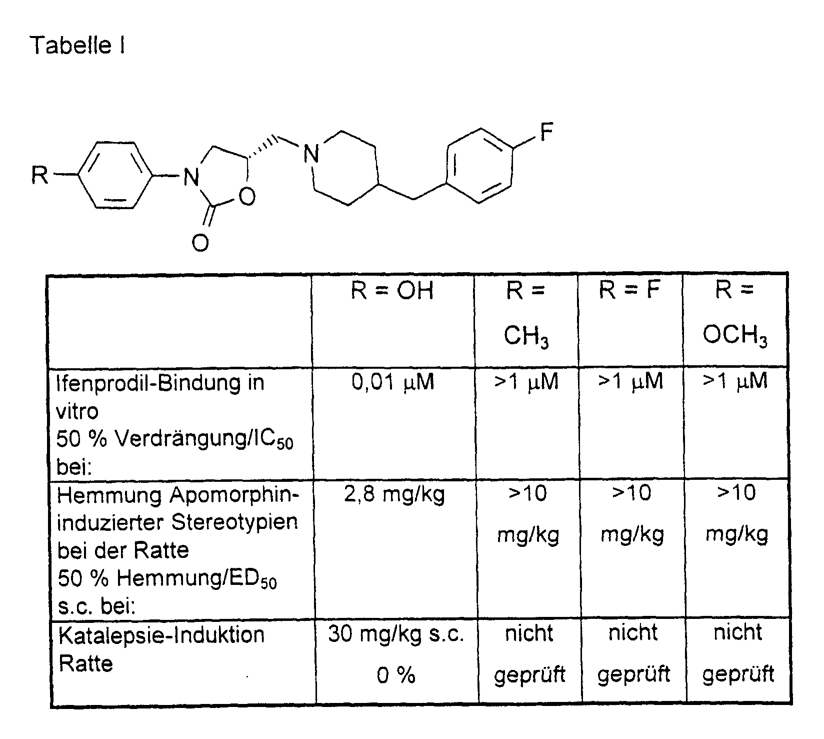

Die getesteten Verbindungen wurden in Form ihrer Methansulfonat-salze eingesetzt.

Die Verbindung (5S)-(-)-5-[4-(4-Fluorbenzyl)-1-piperidinylmethyl]-3-(4-hydroxyphenyl)-oxazolidin-2-on ist in vitro ein potenter Ligand im nanomolaren Konzentrationsbereich (IC₅₀ = 10 nM) für die Polyamin-Bindungsstelle, die eine modulierende Bindungsstelle für den N-Methyl-D-Aspartat Subtyp der Glutamat-Bindungsstellen darstellt.
Die Verbindung hemmt die Apomorphin-induzierten Stereotypien bei der Ratte (ED₅₀ 2,8 mg/kg s.c.), was als Nachweis für eine neuroleptische Wirkung angesehen wird.
Für die Verbindung wird angenommen, daß es keine extrapyramidalen Nebenwirkungen hervorruft, da es in entsprechenden Tiermodellen in vivo keine Katalepsie hervorruft (ED₅₀ >>30 mg/kg).
Die Verbindung stellt ein neues Wirkprinzip zur Behandlung der Schizophrenie dar.

Die nachfolgenden Beispiele betreffen pharmazeutische Zubereitungen:

### Beispiel A: Injektionsgläser

Eine Lösung von 100 g eines Wirkstoffs der Formel I und 5 g Dinatriumhydrogenphosphat wird in 3 l zweifach destilliertem Wasser mit 2 n Salzsäure auf pH 6.5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffs der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffs der Formel I, 9.38 g NaH₂PO₄^{.}2H₂O, 28.48 g Na₂HPO₄·12H₂O und 0.1 g Benzalkoniumchlorid in 940 ml zweifach destilliertem Wasser. Man stellt auf pH 6.8 ein, füllt auf 1 I auf und sterilisiert durch Bestrahlung.

### Beispiel D: Salbe

Man mischt 500 mg eines Wirkstoffs der Formel I mit 99.5 g Vaseline unter aseptischen Bedingungen.

### Beispiel E: Tabletten

Ein Gemisch von 1 kg Wirkstoff der Formel I, 4 kg Laktose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragent und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Die Verbindung 5-[4-(4-Fluorobenzyl)-piperidin-1-ylmethyl]-3-(4-hydroxyphenyl)-oxazolidin-2-on der Formel I 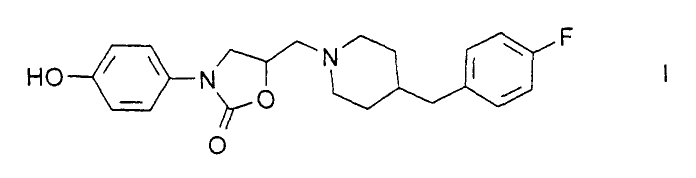 sowie deren physiologisch unbedenklichen Salze.

2. Enantiomere der Verbindungen der Formel I gemäß Anspruch 1.

3. Verfahren zur Herstellung der Verbindung der Formel I gemäß Anspruch 1, **dadurch gekennzeichnet, daß** man
a) eine Verbindung der Formel II 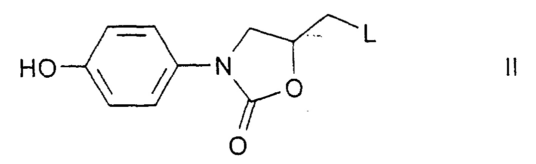 worin
L Cl, Br, I oder eine freie oder reaktionsfähig funktionell abgewandelte OH-Gruppe bedeutet,
mit einer Verbindung der Formel III 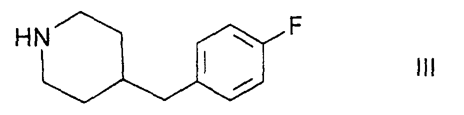 umsetzt,
oder
b) eine Verbindung der Formel IV 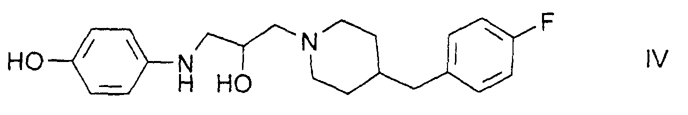 mit einer Verbindung der Formel V 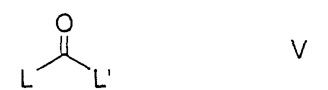 worin L und L'jeweils unabhängig voneinander Cl, Br, I oder eine freie oder reaktionsfähig funktionell abgewandelte OH-Gruppe bedeutet,
umsetzt,
oder
c) eine Verbindung der Formel VI 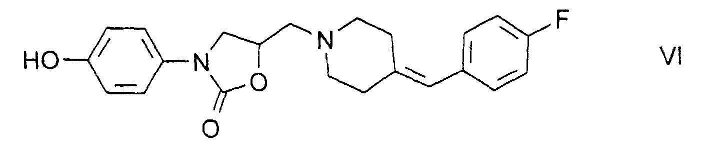 hydriert, oder
d) die Verbindung der Formel I aus einem ihrer funktionellen Derivate durch Behandeln mit einem solvolysierenden oder hydrogenolysierenden Mittel in Freiheit setzt,
und/oder daß man eine basische Verbindung der Formel I durch Behandeln mit einer Säure in eines ihrer Salze überführt.

4. Verfahren zur Herstellung pharmazeutischer Zubereitungen, **dadurch gekennzeichnet, daß** man die Verbindung der Formel I gemäß Anspruch 1 und/oder eines ihrer physiologisch unbedenklichen Salze zusammen mit mindestens einem festen, flüssigen oder halbflüssigen Träger- und/oder Hilfsstoff in eine geeignete Dosierungsform bringt.

5. Pharmazeutische Zubereitung, **gekennzeichnet durch** einen wirksamen Gehalt der Verbindung der Formel I gemäß Anspruch 1 und/oder einem ihrer physiologisch unbedenklichen Salze.

6. Verwendung der Verbindung der Formel I nach Anspruch 1 oder von deren physiologisch unbedenklichen Salzen zur Herstellung eines Arzneimittels.

7. Verbindung der Formel I und ihre physiologisch unbedenklichen Salze als Neuroleptikum zur Bekämpfung von Schizophrenie, affektiven Störungen oder Schlaganfall.

8. Verwendung der Verbindung der Formel I nach Anspruch 1 oder von deren physiologisch unbedenklichen Salzen zur Herstellung von Arzneimitteln mit psychopharmakologischer Wirkung.

9. Verwendung der Verbindung der Formel I nach Anspruch 1 oder von deren physiologisch unbedenklichen Salzen zur Herstellung von Arzneimitteln mit neuroleptischer Wirkung.

## Claims

1. The compound 5-[4-(4-fluorobenzyl)piperidin-1-ylmethyl]-3-(4-hydroxyphenyl)oxazolidin-2-one of the formula I 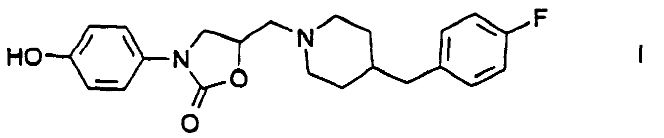 and the physiologically acceptable salts thereof.

2. Enantiomers of the compounds of the formula I according to Claim 1.

3. Process for preparing the compound of the formula I according to Claim 1, **characterized in that**
a) a compound of the formula II 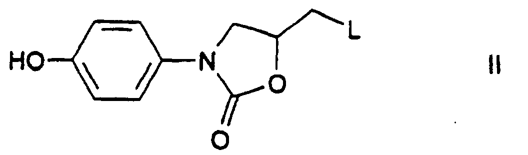 in which
L is Cl, Br, I or a free or reactively functionally modified OH group
is reacted with a compound of the formula III 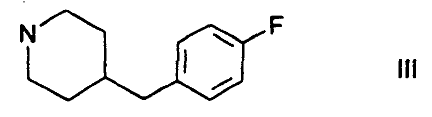 or
b) a compound of the formula IV 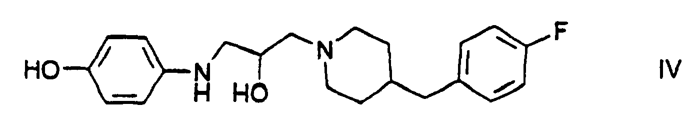 is reacted with a compound of the formula V 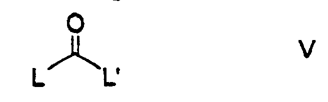 in which L and L' independently of one another are in each case Cl, Br, I or a free or reactively functionally modified OH group
or
c) a compound of the formula VI 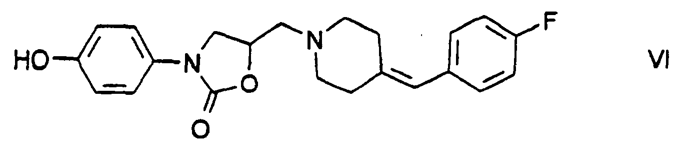 is hydrogenated or
d) the compound of the formula I is liberated from one of its functional derivatives by treatment with a solvolysing or hydrogenolysing agent
and/or **in that** a basic compound of the formula I is converted into one of its salts by treatment with an acid.

4. Process for preparing pharmaceutical preparations, **characterized in that** the compound of the formula I according to Claim 1 and/or one of its physiologically acceptable salts is converted together with at least one solid, liquid or semi-liquid carrier and/or auxiliary into a suitable dosage form.

5. Pharmaceutical preparation, **characterized in that** it contains an effective amount of the compound of the formula I according to Claim 1 and/or one of its physiologically acceptable salts.

6. Use of the compound of the formula I according to Claim 1 or of physiologically acceptable salts thereof for the production of a medicament.

7. Compound of the formula I and its physiologically acceptable salts as neuroleptic for the control of schizophrenia, affective disorders or stroke.

8. Use of the compound of the formula I according to Claim 1 or of physiologically acceptable salts thereof for the production of medicaments having psychopharmacological action.

9. Use of the compound of the formula I according to Claim 1 or of physiologically acceptable salts thereof for the production of medicaments having neuroleptic action.

## Revendications

1. Composé 5-[4-(4-fluorobenzyl)-pipéridin-1-ylméthyl]-3-(4-hydroxyphényl)-oxazolidin-2-one de formule I 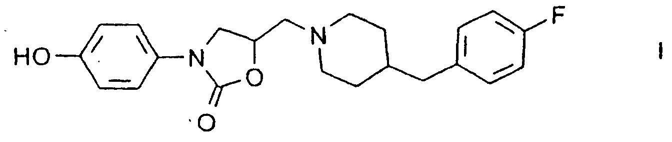 ainsi que ses sels physiologiquement acceptables.

2. Enantiomères des composés de formule I selon la revendication 1.

3. Procédé pour la préparation du composé de formule I selon la revendication 1, **caractérisé en ce que**
a) on fait réagir un composé de formule II 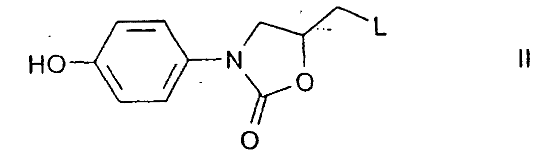 dans laquelle
L représente Cl, Br, I ou un groupe OH libre ou modifié à fonction réactive, avec un composé de formule III 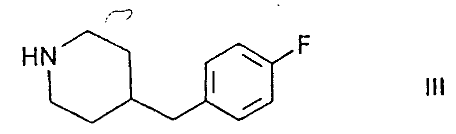 ou
b) on fait réagir un composé de formule IV 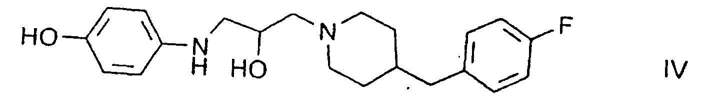 avec un composé de formule V 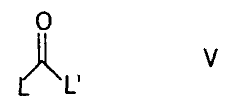 dans laquelle L et L' représentent chacun, indépendamment l'un de l'autre, Cl, Br, I ou un groupe OH libre ou modifié à fonction réactive,
ou
c) on soumet à une hydrogénation un composé de formule VI 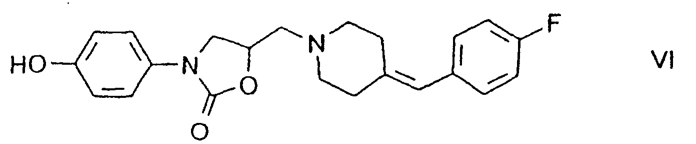 ou
d) on libère le composé de formule I à partir d'un de ses dérivés fonctionnels, par traitement par un agent de solvolyse ou d'hydrogénolyse,
et/ou **en ce qu'**on convertit un composé basique de formule I en un de ses sels par traitement par un acide.

4. Procédé pour la préparation de compositions pharmaceutiques, **caractérisé en ce qu'**on met sous une forme pharmaceutique appropriée le composé de formule I selon la revendication 1 et/ou un de ses sels physiologiquement acceptables, conjointement avec au moins un véhicule et/ou adjuvant solide, liquide ou semi-liquide.

5. Composition pharmaceutique, **caractérisée par** une teneur efficace en le composé de formule I selon la revendication 1 et/ou en un de ses sels physiologiquement acceptables

6. Utilisation du composé de formule I selon la revendication 1 ou de ses sels physiologiquement acceptables, pour la fabrication d'un médicament.

7. Utilisation du composé de formule I et de ses sels physiologiquement acceptables, en tant que neuroleptique pour le traitement de la schizophrénie, de troubles affectifs ou d'un accident cérébral.

8. Utilisation du composé de formule I selon la revendication 1 ou de ses sels physiologiquement acceptables, pour la fabrication d'un médicament à effet psychopharmacologique.

9. Utilisation du composé de formule I selon la revendication 1 ou de ses sels physiologiquement acceptables, pour la fabrication d'un médicament à effet neuroleptique.
